# EUROPEAN PATENT APPLICATION

(11) **EP 4 541 803 A1**
(43) Date of publication of application: **23.04.2025**
(21) Application number: 23823265.6
(22) Date of filing: 16.06.2023
(51) Int. Cl.: C07H 19/167, A61K 31/7076, A61P 29/00

(54) **CYCLOPENTYL ADENOSINE DERIVATIVE AND PHARMACEUTICAL USE THEREOF**

(30) Priority: 17.06.2022 CN 202210691675; 12.10.2022 CN 202211248227
(71) Applicant: Shanghai Senhui Medicine Co., Ltd., Shanghai 201203 (CN); Shanghai Fourth People's Hospital, Shanghai 200434 (CN); Jiangsu Hengrui Pharmaceuticals Co., Ltd., Lianyungang, Jiangsu 222047 (CN)
(72) Inventor: XIONG, Lize, Shanghai 200434 (CN); ZHU, Lingjian, Shanghai 201203 (CN); LI, Wanrong, Shanghai 200434 (CN); JI, Changjin, Shanghai 201203 (CN); HUANG, Jian, Shanghai 201203 (CN)
(74) Representative: Regimbeau
(86) International application number: PCT/CN2023/100742
(87) International publication number: WO 2023/241699

(57) **Abstract**

The present disclosure relates to a cyclopentyl adenosine derivative and a pharmaceutical use thereof. Specifically, the present disclosure provides a compound represented by formula (I) or a pharmaceutically acceptable salt thereof, wherein R¹-R⁸ are as defined in the description.

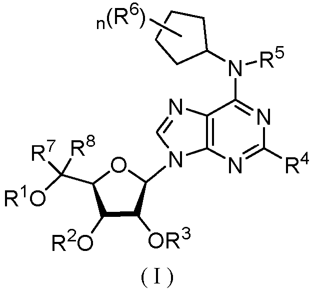

## Description

### TECHNICAL FIELD

The disclosure pertains to the field of pharmaceutics and relates to a cyclopentyl adenosine derivative and pharmaceutical use thereof.

### BACKGROUND

Chronic pain can be categorized by physiological mechanisms into nociceptive pain (somatic and visceral) and non-nociceptive pain (neuropathic and psychogenic), and its clinical manifestations include tissue injury pain and neurogenic pain. Adenosine is a precursor and a metabolite of adenine nucleotides, and its receptors include A₁, A₂A, A₂B, and A₃ receptors. The A₁ receptor plays a key role in pain transmission; it activates G protein-coupled receptors (GPCRs) to regulate the transmembrane flow of cations in cells, affecting neuronal excitability and transmitter release, thereby providing analgesic, anti-inflammatory, etc. effects.

Patent No. US6110902A discloses related inspiration to use an adenosine A₁ receptor agonist CCPA (2-chloro-N-cyclopentyladenosine) to treat chronic pain. However, although the A₁ receptor agonist has shown good clinical effects on chronic pain, its cardiovascular side effects and narrow therapeutic window limit its use. Therefore, developing an adenosine A₁ receptor agonist with a wider therapeutic window and lower cardiovascular risk is now an urgent technical problem that needs to be addressed.

### SUMMARY

The disclosure provides a compound represented by formula (I) or a pharmaceutically acceptable salt thereof, 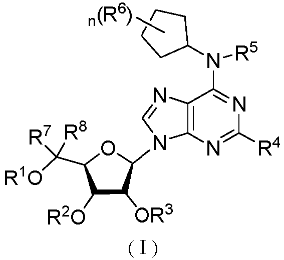 wherein:
R¹, R², and R³ are identical or different and are each independently selected from the group consisting of hydrogen, 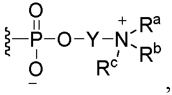 -(CO)-C₁₋₂₀ alkyl, -(CO)-C₁₋₂₀ alkoxy, - (CO)-5-6 membered cycloalkyl, -(CO)-5-6 membered heterocyclyl, -(CO)-6-membered aryl, and -(CO)-5-6 membered heteroaryl, wherein the alkyl, alkoxy, cycloalkyl, heterocyclyl, aryl, or heteroaryl is optionally substituted with one or more substituents selected from the group consisting of halogen, hydroxy, oxo, amino, cyano, C₁₋₆ alkyl, and C₁₋₆ alkoxy;
Y is selected from the group consisting of alkylene and heteroalkylene, wherein the alkylene or heteroalkylene is optionally substituted with one or more substituents selected from the group consisting of halogen, hydroxy, oxo, amino, cyano, C₁₋₆ alkyl, and C₁₋₆ alkoxy;
R^{a}, R^{b}, and R^{c} are identical or different and are each independently C₁₋₆ alkyl,
and R¹, R², and R³ cannot be hydrogen at the same time;
R⁴ is selected from the group consisting of halogen, hydroxy, amino, cyano, C₁₋₆ alkyl, and C₁₋₆ alkoxy, wherein the alkyl or alkoxy is optionally substituted with one or more substituents selected from the group consisting of halogen, hydroxy, amino, cyano, C₁₋₆ alkyl, and C₁₋₆ alkoxy;
R⁵, R⁶, R⁷, and R⁸ are identical or different and are each independently selected from the group consisting of hydrogen, halogen, hydroxy, amino, cyano, C₁₋₆ alkyl, and C₁₋₆ alkoxy, wherein the alkyl or alkoxy is optionally substituted with one or more substituents selected from the group consisting of halogen, hydroxy, amino, cyano, C₁₋₆ alkyl, and C₁₋₆ alkoxy;
n is selected from the group consisting of 0, 1, 2, 3, 4, 5, 6, 7, and 8.

In some embodiments, provided is the compound represented by formula (I) or the pharmaceutically acceptable salt thereof, wherein both R² and R³ are hydrogens.

In some embodiments, provided is the compound or the pharmaceutically acceptable salt thereof according to the disclosure, wherein the compound represented by formula (I) or the pharmaceutically acceptable salt thereof is a compound represented by formula (I-1) or a pharmaceutically acceptable salt thereof, 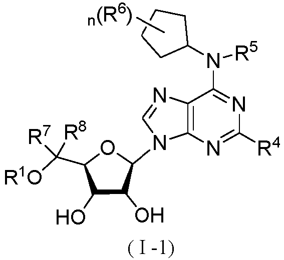 wherein R¹, R⁴, R⁵, R⁶, R⁷, R⁸, and n are as defined in formula (I).

In some embodiments, provided is the compound represented by formula (I) or (I-1) or the pharmaceutically acceptable salt thereof according to the disclosure, wherein R⁴ is halogen, preferably fluorine, chlorine, or bromine, and most preferably chlorine.

In some embodiments, provided is the compound or the pharmaceutically acceptable salt thereof according to the disclosure, wherein the compound represented by formula (I) or the pharmaceutically acceptable salt thereof is a compound represented by formula (I-2) or a pharmaceutically acceptable salt thereof, 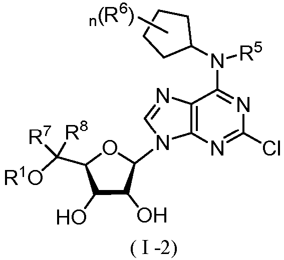 wherein R¹, R⁵, R⁶, R⁷, R⁸, and n are as defined in formula (I).

In some embodiments, provided is the compound represented by formula (I), formula (I-1), or formula (I-2) or the pharmaceutically acceptable salt thereof according to the disclosure, wherein R⁵ is hydrogen.

In some embodiments, provided is the compound represented by formula (I), formula (I-1), or formula (I-2) or the pharmaceutically acceptable salt thereof according to the disclosure, wherein R⁶ is hydrogen.

In some embodiments, provided is the compound or the pharmaceutically acceptable salt thereof according to the disclosure, wherein the compound represented by formula (I) or the pharmaceutically acceptable salt thereof is a compound represented by formula (I-3) or a pharmaceutically acceptable salt thereof, 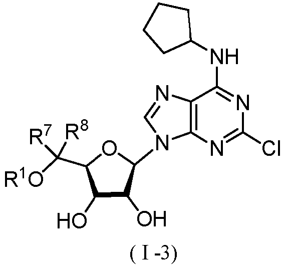 wherein R¹, R⁷, and R⁸ are as defined in formula (I).

In some embodiments, provided is the compound represented by formula (I), formula (I-1), formula (I-2), or formula (I-3) or the pharmaceutically acceptable salt thereof according to the disclosure, wherein both R⁷ and R⁸ are hydrogens.

In some embodiments, provided is the compound or the pharmaceutically acceptable salt thereof according to the disclosure, wherein the compound represented by formula (I) or the pharmaceutically acceptable salt thereof is a compound represented by formula (I-4) or a pharmaceutically acceptable salt thereof, 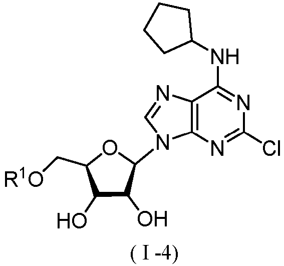 wherein R¹ is as defined in formula (I).

In some embodiments, provided is the compound represented by formula (I), formula (I-1), formula (I-2), formula (I-3), or formula (I-4) or the pharmaceutically acceptable salt thereof according to the disclosure, wherein R¹ is selected from the group consisting of - (CO)-C₁₋₅ alkyl, -(CO)-C₁₀₋₂₀ alkyl, -(CO)-5-6 membered cycloalkyl, -(CO)-5-6 membered heterocyclyl, -(CO)-phenyl, -(CO)-5-6 membered heteroaryl, and 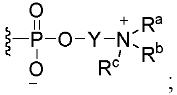
Y is C₁₋₆ alkylene;
R^{a}, R^{b}, and R^{c} are identical or different and are each independently C₁₋₆ alkyl;
the alkylene, alkyl, cycloalkyl, heterocyclyl, phenyl, or heteroaryl is optionally substituted with one or more substituents selected from the group consisting of halogen, hydroxy, amino, cyano, oxo, C₁₋₆ alkyl, and C₁₋₆ alkoxy, and the substituents are preferably fluorine, chlorine, bromine, hydroxy, amino, oxo, and methyl.

In some embodiments, provided is the compound represented by formula (I), formula (I-1), formula (I-2), formula (I-3), or formula (I-4) or the pharmaceutically acceptable salt thereof according to the disclosure, wherein R¹ is selected from the group consisting of - (CO)-methyl, -(CO)-ethyl, -(CO)-propyl, -(CO)-butyl, -(CO)-pentyl, -(CO)-C₁₄ alkyl, - (CO)-phenyl, -(CO)-cyclopropane, -(CO)-cyclobutane, -(CO)-cyclopentane, -(CO)-cyclohexane, -(CO)-pyrrolidine, -(CO)-piperidine, -(CO)-piperazine, -(CO)-morpholine, -(CO)-thiomorpholine, -(CO)-pyrrole, -(CO)-furan, -(CO)-thiophene, -(CO)-pyridine, - (CO)-thiazole, -(CO)-oxazole, -(CO)-imidazole, -(CO)-triazole, -(CO)-tetrazole, and 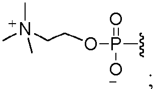 the R¹ is optionally substituted with one or more substituents selected from the group consisting of halogen, hydroxy, amino, cyano, oxo, C₁₋₆ alkyl, and C₁₋₆ alkoxy, and the substituents are preferably fluorine, chlorine, bromine, hydroxy, amino, oxo, and methyl. In some embodiments, provided is the compound represented by formula (I), formula (I-1), formula (I-2), formula (I-3), or formula (I-4) or the pharmaceutically acceptable salt thereof according to the disclosure, wherein R¹ is selected from the group consisting of - (CO)-C₁₀ alkyl, -(CO)-C₁₁ alkyl, -(CO)-C₁₂ alkyl, -(CO)-C₁₃ alkyl, -(CO)-C₁₄ alkyl, - (CO)-C₁₅ alkyl, -(CO)-C₁₆ alkyl, -(CO)-C₁₇ alkyl, and -(CO)-C₁₈ alkyl; further, the R¹ is optionally substituted with one or more substituents selected from the group consisting of halogen, hydroxy, amino, cyano, oxo, C₁₋₆ alkyl, and C₁₋₆ alkoxy, and the substituents are preferably fluorine, chlorine, bromine, hydroxy, amino, oxo, and methyl.

In some embodiments, provided is the compound represented by formula (I), formula (I-1), formula (I-2), formula (I-3), or formula (I-4) or the pharmaceutically acceptable salt thereof according to the disclosure, wherein R¹ is selected from the group consisting of - (CO)-C₁₂ alkyl, -(CO)-C₁₃ alkyl, -(CO)-C₁₄ alkyl, -(CO)-C₁₅ alkyl, -(CO)-C₁₆ alkyl; further, the R¹ is optionally substituted with one or more substituents selected from the group consisting of halogen, hydroxy, amino, cyano, oxo, C₁₋₆ alkyl, and C₁₋₆ alkoxy, and the substituents are preferably fluorine, chlorine, bromine, hydroxy, amino, oxo, and methyl.

In some embodiments, specific compounds of the disclosure include, but are not limited to,

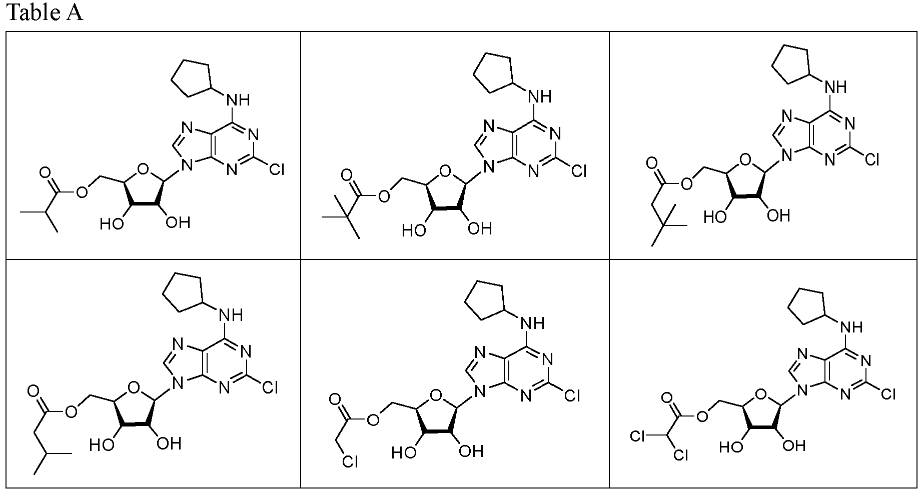

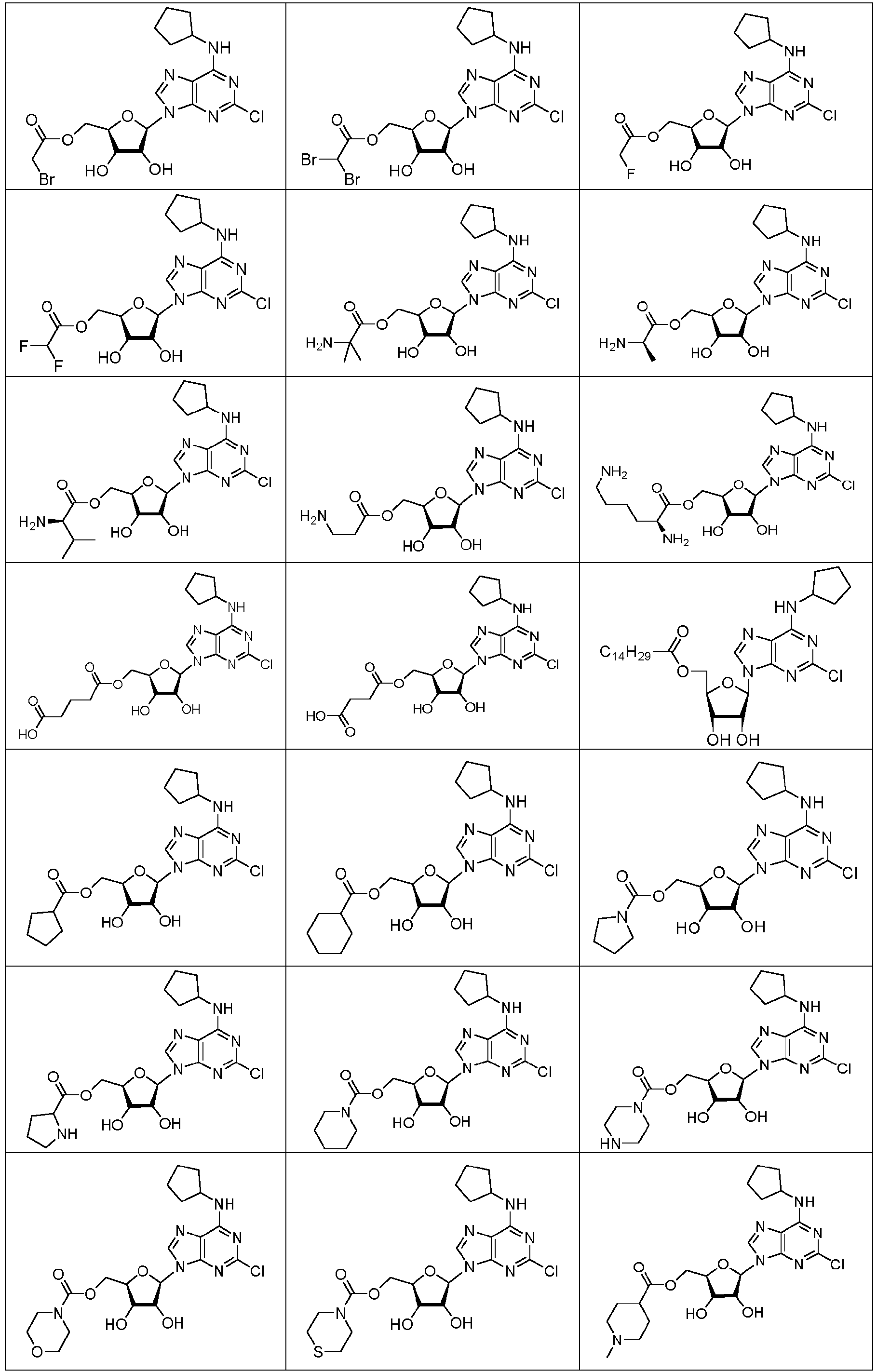

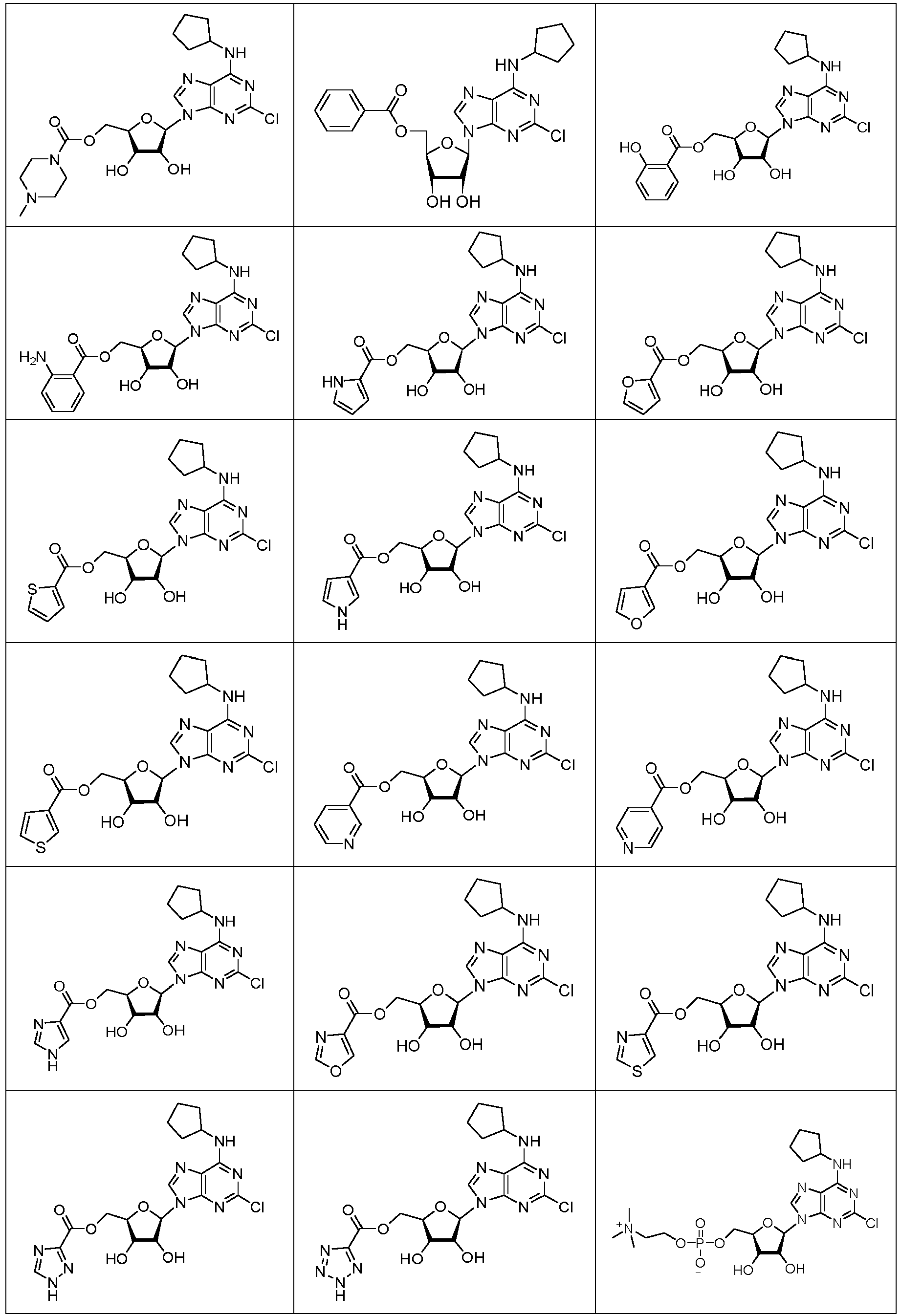

The disclosure further provides isotopically substituted forms of the aforementioned compounds or the pharmaceutically acceptable salts thereof. In some embodiments, the isotopically substituted forms are deuterated forms.

The disclosure further provides a pharmaceutical composition comprising a therapeutically effective amount of at least one of the aforementioned compounds represented by formula (I), formula (I-1), formula (I-2), formula (I-3), and formula (I-4) and the compounds shown in Table A or a pharmaceutically acceptable salt, or an isotopically substituted form thereof, and a pharmaceutically acceptable excipient.

In some embodiments, a unit dose of the pharmaceutical composition is 0.001 mg-1000 mg.

In certain embodiments, the pharmaceutical composition comprises 0.01-99.99% of an aforementioned compound or a pharmaceutically acceptable salt thereof or an isotopically substituted form thereof based on the total weight of the composition. In certain embodiments, the pharmaceutical composition comprises 0.1-99.9% of an aforementioned compound or a pharmaceutically acceptable salt thereof or an isotopically substituted form thereof. In certain embodiments, the pharmaceutical composition comprises 0.5%-99.5% of an aforementioned compound or a pharmaceutically acceptable salt thereof or an isotopically substituted form thereof. In certain embodiments, the pharmaceutical composition comprises 1%-99% of an aforementioned compound or a pharmaceutically acceptable salt thereof or an isotopically substituted form thereof. In certain embodiments, the pharmaceutical composition comprises 2%-98% of an aforementioned compound or a pharmaceutically acceptable salt thereof or an isotopically substituted form thereof.

In certain embodiments, the pharmaceutical composition comprises 0.01%-99.99% of the pharmaceutically acceptable excipient based on the total weight of the composition. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

The compounds or the pharmaceutically acceptable salts thereof or the isotopically substituted forms thereof provided by the disclosure or the aforementioned pharmaceutical composition may be used for ameliorating all forms of acute and chronic pain, particularly pain caused by cancer, neurogenic pain, post-operative pain, neuropathic pain, and neuralgia.

The compounds or the pharmaceutically acceptable salts thereof or the isotopically substituted forms thereof provided by the disclosure or the aforementioned pharmaceutical composition may also be used for treating a particular pain syndrome, including: pain associated with soft tissue diseases and peripheral injuries (e.g., acute trauma, osteoarthritis, rheumatoid arthritis, burns, and episiotomy), spinal pain, musculoskeletal pain, upper limb pain, limb pain, myofascial pain syndrome, headache, deep and visceral pain syndrome (e.g., cardiac pain, muscular pain, eye pain, orofacial pain, abdominal pain, gynecological pain, and labor pain), pain associated with nerve and root injuries (e.g., peripheral nerve disease or infection, pain caused by amputation, peripheral neuropathy, twitch and atypical facial pain, and arachnoiditis), cancer pain (particularly pain related to bone and soft tissue cancer and metastasis), and central nervous system pain (e.g., central pain caused by spinal cord or brainstem injury).

The disclosure further provides a method for treating or preventing chronic pain, comprising administering to a patient a therapeutically effective amount of the aforementioned compound represented by formula (I), formula (I-1), formula (I-2), formula (I-3), or formula (I-4) or a compound shown in Table A or a pharmaceutically acceptable salt thereof or an isotopically substituted form thereof, or the aforementioned pharmaceutical composition.

The disclosure further provides use of the aforementioned compound represented by formula (I), formula (I-1), formula (I-2), formula (I-3), or formula (I-4) or a compound shown in Table A or a pharmaceutically acceptable salt thereof or the aforementioned pharmaceutical composition in the preparation of a medicament for treating or preventing chronic pain. In some embodiments, the chronic pain includes, but is not limited to, tissue injury pain, neurogenic pain, headache, migraine, tension headache, cluster headache, chronic tension-type headache, inflammatory pain, neck and shoulder pain, low back and leg pain, neuropathic pain, eye pain, and dental pain.

The pharmaceutically acceptable salts of the compounds according to the disclosure may be selected from the group consisting of inorganic salts and organic salts.

The compounds of the disclosure may have particular geometric or stereoisomeric forms. The disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which are within the scope of the disclosure. Additional asymmetric carbon atoms may be present in substituents such as an alkyl group. All such isomers and mixtures thereof are included within the scope of the disclosure. The compounds of the disclosure containing asymmetric carbon atoms may be separated in optically active pure form or in racemic form. The optically active pure form may be isolated from a racemic mixture or synthesized using chiral starting materials or chiral reagents.

Optically active (R)- and (S)-enantiomers and D- and L-isomers can be prepared by chiral synthesis, chiral reagents, or other conventional techniques. If one enantiomer of a certain compound of the disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, followed by resolution of diastereomers by conventional methods known in the art, and the pure enantiomers are obtained by recovery. In addition, separation of enantiomers and diastereomers is generally accomplished by chromatography using a chiral stationary phase, optionally in combination with chemical derivatization (e.g., carbamate formation from amines).

In the chemical structures of the compounds according to the disclosure, a bond " 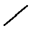 " represents an unspecified configuration; that is, if chiral isomers exist in the chemical structures, the bond " 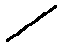 " may be " 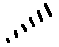 " or " 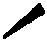 ", or both the configurations of " 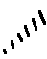 " and " 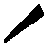 " are included simultaneously.

In the chemical structures of the compounds according to the disclosure, a bond " 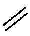 " is not specified with a configuration; that is, they may be in a Z configuration or an E configuration, or contain both configurations.

The compounds and intermediates of the disclosure may also exist in different tautomeric forms, and all such forms are included within the scope of the disclosure. The term "tautomer" or "tautomeric form" refers to structural isomers of different energies that can interconvert via a low-energy barrier. For example, proton tautomers (also known as proton transfer tautomers) include interconversion via proton migration, such as ketoenol and imine-enamine isomerization. An example of a lactam-lactim equilibrium is present between A and B as shown below.

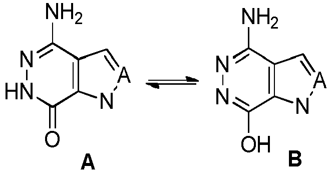

All compounds in the disclosure can be drawn as form A or form B. All tautomeric forms are within the scope of the disclosure. The names of the compounds do not exclude any tautomers.

The disclosure also includes isotopically labeled compounds that are identical to those recited herein but have one or more atoms replaced by an atom having an atomic mass or mass number different from the atomic mass or mass number usually found in nature. Examples of isotopes that can be incorporated into the compounds of the disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Unless otherwise specified, when a position is specifically assigned deuterium (D), the position should be construed as deuterium with an abundance that is at least 1000 times greater than the natural abundance of deuterium (which is 0.015%) (i.e., at least 10% deuterium incorporation). The compounds of examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The disclosure also includes various deuterated forms of the compound of formula (I). Each available hydrogen atom linked to a carbon atom may be independently replaced by a deuterium atom. Those skilled in the art are able to synthesize the deuterated forms of the compound of formula (I) with reference to the relevant literature. Commercially available deuterated starting materials can be used in preparing the deuterated forms of the compound of formula (I), or they can be synthesized using conventional techniques with deuterated reagents, including but not limited to deuterated borane, tri-deuterated borane in tetrahydrofuran, deuterated lithium aluminum hydride, deuterated iodoethane, deuterated iodomethane, and the like.

"Optionally" or "optional" means that the event or circumstance subsequently described may, but does not necessarily, occur, and this description includes instances where the event or circumstance occurs or does not occur. For example, "C₁₋₆ alkyl that is optionally substituted with a halogen or cyano" means that the halogen or cyano may, but does not necessarily, exist, and this description includes the instance where the alkyl is substituted with a halogen or cyano and the instance where the alkyl is not substituted with a halogen or cyano.

### Terminology

Unless otherwise stated, the terms used in the specification and claims have the following meanings.

"Alkyl" refers to a saturated aliphatic hydrocarbon group, including linear and branched groups of 1 to 20 carbon atoms, and alkyl containing 1 to 6 carbon atoms. Non-limiting examples include methyl, ethyl, n-propyl, isopropyl, n-butyl, isobutyl, tert-butyl, secbutyl, n-pentyl, 1,1-dimethylpropyl, 1,2-dimethylpropyl, 2,2-dimethylpropyl, various branched-chain isomers thereof, and the like. Alkyl may be substituted or unsubstituted, and when it is substituted, the substituent may be substituted at any accessible point of attachment and is preferably one or more groups independently selected from the group consisting of halogen, hydroxy, oxo, amino, C₁₋₆ alkyl, C₁₋₆ alkoxy, 3- to 6-membered cycloalkyl, 3- to 6-membered heterocyclyl, 6- to 10-membered aryl, and 5- to 10-membered heteroaryl, wherein the alkyl, alkoxy, cycloalkyl, heterocyclyl, aryl, or heteroaryl is optionally substituted with one or more halogens, hydroxy, amino, cyano, C₁₋₆ alkyl, or C₁₋₆ alkoxy.

The term "cycloalkyl" or "carbocycle" refers to a saturated or partially unsaturated, monocyclic or polycyclic hydrocarbon substituent. The cycloalkyl ring contains 3 to 20 carbon atoms, preferably 3 to 7 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, and the like. Polycyclic cycloalkyl includes spirocycloalkyl, fused cycloalkyl, and bridged cycloalkyl. Cycloalkyl may be substituted or unsubstituted, and when it is substituted, the substituent may be substituted at any accessible point of attachment and is preferably one or more groups independently selected from the group consisting of halogen, hydroxy, oxo, amino, cyano, C₁₋₆ alkyl, C₁₋₆ alkoxy, 3- to 6-membered cycloalkyl, 3- to 6-membered heterocyclyl, 6- to 10-membered aryl, and 5- to 10-membered heteroaryl, wherein the alkyl, alkoxy, cycloalkyl, heterocyclyl, aryl, or heteroaryl is optionally substituted with one or more halogens, hydroxy, amino, cyano, C₁₋₆ alkyl, or C₁₋₆ alkoxy.

The term "heterocyclyl" or "heterocycloalkyl" refers to a saturated or partially unsaturated monocyclic or polycyclic substituent containing 3 to 20 ring atoms, wherein one or more of the ring atoms are a heteroatom selected from the group consisting of nitrogen, oxygen, and sulfur, and the sulfur may be optionally substituted with oxo (i.e., to form sulfoxide or sulfone), but excluding a ring portion of -O-O-, -O-S-, or -S-S-, and the remaining ring atoms are carbons. It preferably contains 3 to 12 (e.g., 3, 4, 5, 6, 7, 8, 9, 10, 11, and 12) ring atoms, of which 1-4 (e.g., 1, 2, 3, and 4) are heteroatoms; more preferably 3 to 8 (e.g., 3, 4, 5, 6, 7, and 8) ring atoms, of which 1-3 (e.g., 1, 2, and 3) are heteroatoms; more preferably 3 to 6 ring atoms, of which 1-3 are heteroatoms; and most preferably 5 or 6 ring atoms, of which 1-3 are heteroatoms. Non-limiting examples of monocyclic heterocyclyl include pyrrolidinyl, piperidinyl, piperazinyl, N-alkylpiperazinyl, N-alkylpiperidinyl, morpholinyl, thiomorpholinyl, and the like. Polycyclic heterocyclyl includes spiroheterocyclyl, fused heterocyclyl, and bridged heterocyclyl.

Heterocyclyl may be optionally substituted or unsubstituted, and when it is substituted, the substituent is preferably one or more groups independently selected from the group consisting of halogen, hydroxy, oxo, amino, cyano, C₁₋₆ alkyl, C₁₋₆ alkoxy, 3- to 6-membered cycloalkyl, 3- to 6-membered heterocyclyl, 6- to 10-membered aryl, and 5- to 10-membered heteroaryl, wherein the alkyl, alkoxy, cycloalkyl, heterocyclyl, aryl, or heteroaryl is optionally substituted with one or more halogens, hydroxy, amino, cyano, C₁₋₆ alkyl, or C₁₋₆ alkoxy.

The term "aryl" refers to a 6- to 14-membered, preferably 6- to 10-membered all-carbon monocyclic or fused polycyclic (in which the rings share a pair of adjacent carbon atoms) group having a conjugated π-electron system, such as phenyl and naphthyl.

Aryl may be optionally substituted or unsubstituted, and when it is substituted, the substituent is preferably one or more groups independently selected from the group consisting of halogen, hydroxy, oxo, nitro, cyano, amino, C₁₋₆ alkyl, C₁₋₆ alkoxy, 3- to 6-membered cycloalkyl, and 3- to 6-membered heterocyclyl, wherein the alkyl, alkoxy, cycloalkyl, or heterocyclyl is substituted with one or more groups selected from the group consisting of halogen, hydroxy, amino, C₁₋₆ alkyl, and C₁₋₆ alkoxy.

The term "heteroaryl" refers to a heteroaromatic system containing 1 to 4 (e.g., 1, 2, 3, and 4) heteroatoms and 5 to 14 ring atoms, wherein the heteroatoms are selected from the group consisting of oxygen, sulfur, and nitrogen. Heteroaryl is preferably 5- to 10-membered (e.g., 5-membered, 6-membered, 7-membered, 8-membered, 9-membered, or 10-membered) and is more preferably 5-membered or 6-membered, e.g., furyl, thienyl, pyridinyl, pyrrolyl, N-alkylpyrrolyl, imidazolyl, triazolyl, or tetrazolyl.

Heteroaryl may be optionally substituted or unsubstituted, and when it is substituted, the substituent is preferably one or more groups independently selected from the group consisting of halogen, hydroxy, oxo, nitro, cyano, amino, C₁₋₆ alkyl, C₁₋₆ alkoxy, 3- to 6-membered cycloalkyl, and 3- to 6-membered heterocyclyl, wherein the alkyl, alkoxy, cycloalkyl, or heterocyclyl is substituted with one or more groups selected from the group consisting of halogen, hydroxy, amino, C₁₋₆ alkyl, and C₁₋₆ alkoxy.

The term "alkoxy" refers to -O-(alkyl), wherein the alkyl is as defined above. Non-limiting examples of alkoxy include: methoxy, ethoxy, propoxy, butoxy, cyclopropyloxy, cyclobutoxy, cyclopentyloxy, and cyclohexyloxy. Alkoxy may be optionally substituted or unsubstituted, and when it is substituted, the substituent is preferably one or more groups independently selected from the group consisting of halogen, hydroxy, oxo, nitro, cyano, amino, C₁₋₆ alkyl, C₁₋₆ alkoxy, 3- to 6-membered cycloalkyl, and 3- to 6-membered heterocyclyl, wherein the alkyl, alkoxy, cycloalkyl, or heterocyclyl is substituted with one or more groups selected from the group consisting of halogen, hydroxy, amino, C₁₋₆ alkyl, and C₁₋₆ alkoxy.

The term "alkylene" refers to the remainder of an alkane molecule after 2 hydrogen atoms are removed from the alkane molecule, including linear and branched -ylene groups of 1 to 20 carbon atoms. Non-limiting examples of alkylene containing 1 to 6 carbon atoms include methylene (-CH₂-) and ethylene (e.g., -CH₂CH₂- or -CH(CH₃)-). Unless otherwise specified, alkylene may be substituted or unsubstituted.

The term "heteroalkylene" means that one or more -CH₂- in alkylene are replaced with a heteroatom selected from the group consisting of N, O, and S; wherein the alkylene is as defined above; heteroalkylene may be substituted or unsubstituted. The term "hydroxy" refers to the -OH group.

The term "halogen" refers to fluorine, chlorine, bromine, or iodine.

The term "cyano" refers to -CN.

The term "amino" refers to -NH₂.

The term "nitro" refers to -NO₂.

The term "oxo" refers to the =O substituent.

"Substituted" means that one or more, preferably up to 5, and more preferably 1-3, hydrogen atoms in the group are independently substituted with a corresponding number of substituents. It goes without saying that a substituent is only in its possible chemical position, and those skilled in the art will be able to determine (experimentally or theoretically) possible or impossible substitutions without undue effort.

"Pharmaceutical composition" refers to a mixture containing one or more of the compounds or the physiologically/pharmaceutically acceptable salts or pro-drugs thereof described herein, and other chemical components, as well as other components, e.g., physiologically/pharmaceutically acceptable carriers and excipients. The pharmaceutical composition is intended to promote administration to an organism and facilitate the absorption of the active ingredient so that it can exert its bioactivity.

"Pharmaceutically acceptable excipient" includes, but is not limited to, any adjuvant, carrier, glidant, sweetener, diluent, preservative, dye/colorant, flavoring agent, surfactant, wetting agent, dispersant, suspending agent, stabilizer, isotonic agent, solvent, or emulsifier that has been approved by the U.S. Food and Drug Administration as acceptable for use in humans or livestock animals.

The term "pharmaceutically acceptable" means that those compounds, materials, compositions, and/or dosage forms that are, within the scope of reasonable medical judgment, suitable for use in contact with the tissues of patients without excessive toxicity, irritation, allergic reaction, or other problems or complications, and are commensurate with a reasonable benefit/risk ratio and effective for the intended use.

"Effective amount" or "therapeutically effective amount" described in the disclosure includes an amount sufficient to ameliorate or prevent a symptom or disorder of a medical disorder. An effective amount also means an amount sufficient to allow or facilitate diagnosis. The effective amount for a particular patient or veterinary subject may vary depending on factors such as the disorder to be treated, the general health of the patient, the method and route and dosage of administration, and the severity of side effects. An effective amount may be the maximum dose or administration regimen to avoid significant side effects or toxic effects.

For drugs or pharmacologically active agents, the term "therapeutically effective amount" refers to an amount of a drug or an agent that is sufficient to provide the desired effect but is non-toxic. The determination of the effective amount varies from person to person. It depends on the age and general condition of a subject, as well as the specific active substance used. The appropriate effective amount in a case may be determined by those skilled in the art in the light of routine tests.

As used herein, the singular forms "a", "an" and "the" include plural references and vice versa, unless otherwise clearly defined in the context.

The compounds according to the disclosure may be prepared according to the following general reaction scheme or similar methods. However, the conditions for the methods, such as reactants, solvents, bases, the amount of the compound used, reaction temperature, and time required for the reaction, are not limited to the explanations in the prior art. The compounds of the disclosure may also be conveniently prepared by optionally combining various synthesis methods described in the specification or known in the art, and such combinations may be readily obtained by those skilled in the art.

### DETAILED DESCRIPTION

The disclosure is further described below with reference to examples. However, these examples do not limit the scope of the disclosure.

Experimental methods without conditions specified in the examples of the disclosure were generally conducted under conventional conditions, or conditions recommended by the manufacturers of the starting materials or commercial products. Reagents without specific origins indicated were commercially available conventional reagents.

The structures of the compounds were determined by nuclear magnetic resonance (NMR) spectroscopy or/and mass spectrometry (MS). The NMR shifts (δ) are given in 10⁻⁶ (ppm). The NMR analyses were performed on a Bruker AVANCE-400 nuclear magnetic resonance instrument, with dimethyl sulfoxide-D6 (DMSO-*d₆*), chloroform-D (CDCl₃), and methanol-D4 (methanol-*d₄*) as solvents and tetramethylsilane (TMS) as an internal standard.

The HPLC analyses were performed using an Agilent1100 high pressure liquid chromatograph, a GAS15B DAD ultraviolet detector, and a Water Vbridge C18 150 × 4.6 mm 5 µm chromatography column.

The MS analyses were performed using an Agilent6120 triple quadrupole mass spectrometer, a G1315D DAD detector, and a Waters Xbridge C18 4.6 × 50 mm, 5 µm chromatography column, with scanning performed in positive/negative ion mode with a mass scan range of 80-1200.

The thin-layer chromatography silica gel plates used were Yantai Huanghai HSGF254 silica gel plates. The silica gel plates used in the thin-layer chromatography (TLC) analyses had a layer thickness of 0.2 mm ± 0.03 mm, and those used in the thin-layer chromatography separation and purification had a layer thickness of 0.4 mm-0.5 mm.

The flash column purification system used was Combiflash Rf150 (TELEDYNE ISCO) or Isolara one (Biotage).

In the normal-phase column chromatography steps, a 200-300 mesh or 300-400 mesh Yantai Huanghai silica gel was generally used as the carrier, or a Changzhou Santai prefill ultrapure normal-phase silica gel column (40-63 µm, 60 g, 24 g, 40 g, 120 g, or other specifications) was used.

The known starting materials in the disclosure may be synthesized by using or following methods known in the art, or may be purchased from Shanghai Titan Scientific, ABCR GmbH & Co. KG, Acros Organics, Aldrich Chemical Company, Accela ChemBio Inc., and Bide Pharmatech, among others.

In the examples, the reactions can all be performed in a nitrogen atmosphere unless otherwise specified.

The nitrogen atmosphere means that the reaction flask is connected to a balloon containing about 1 L of nitrogen gas.

The hydrogen atmosphere means that the reaction flask was connected to a balloon containing about 1 L of hydrogen gas.

The hydrogen gas was prepared by a QPH-1L hydrogen generator from Shanghai Quan Pu Scientific Instruments Inc.

The nitrogen atmosphere or the hydrogenation atmosphere was generally formed by 3 cycles of vacuumization and nitrogen or hydrogen filling.

In the examples, the solutions were aqueous solutions unless otherwise specified.

In the examples, the reaction temperature was room temperature, i.e., 20 °C-30 °C, unless otherwise specified.

The monitoring of reaction progress in the examples was performed using thin-layer chromatography (TLC). For the developing solvents used in the reactions, the eluent systems used in the column chromatography purification, and the developing solvent systems used in the thin-layer chromatography analyses, the volume ratio of the solvents was adjusted depending on the polarity of the compound, or by adding a small amount of basic or acidic reagents such as triethylamine and acetic acid.

### Examples

### Example 1: ((2R,3S,4R,5R)-5-(2-Chloro-6-(cyclopentylamino)-9H-purin-9-yl)-3,4-dihydroxytetrahydrofuran-2-yl)methyl pentadecanoate (Compound 1)

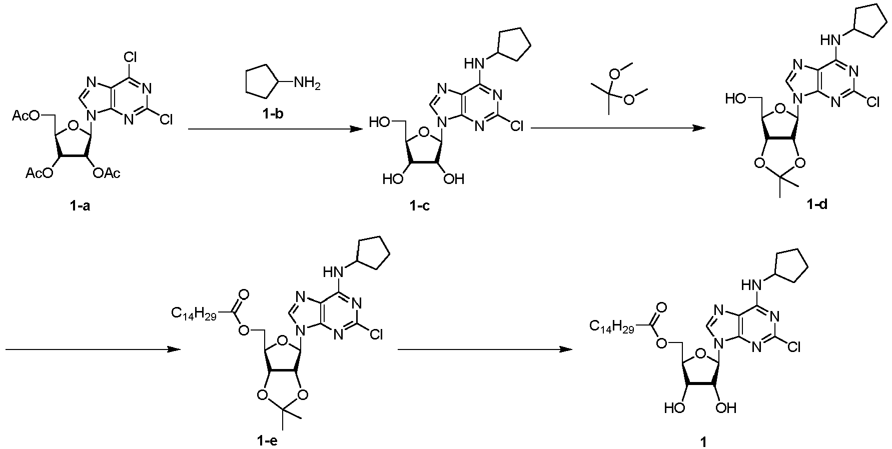

### Step 1) Preparation of (2R,3R,4S,5R)-2-(2-chloro-6-(cyclopentylamino)-9H-purin-9-yl)-5-(hydroxymethyl)tetrahydrofuran-3,4-diol (1-c)

**1-a** (6.0 g, 13.4 mmol), ethanol (40 mL), **1-b** (1.370 g, 16.1 mmol), and TEA (2.2 mL, 16.1 mmol) were sequentially added to a 250 mL three-necked flask. Stirring was started, and the system was purged with nitrogen gas three times and heated at reflux (internal temperature: 70 °C) for 2 h. Analysis showed that the reaction of the starting material was complete, and the reaction was stopped and cooled until the internal temperature was 20 °C. The reaction mixture was concentrated under reduced pressure to give an oil. Methanol (120 mL) and potassium carbonate (12.9 g, 93.4 mmol) were sequentially added, and the mixture was stirred at room temperature for 3 h. The reaction was stopped, and the reaction mixture was concentrated under reduced pressure to give an oil. Water (50 mL), saturated ammonium chloride (150 mL), and dichloromethane were sequentially added. Extraction was performed with dichloromethane three times, 60 mL each time. The organic phases were combined, extracted and washed with saturated brine (50 mL), dried over anhydrous sodium sulfate (5 g), filtered, and concentrated to give an oil. The oil was purified by column chromatography (MeOH/DCM = 0-10%) to give a yellow solid (4.750 g, yield: 95.9%).

MS-ESI: 370.1[M+H]⁺.

¹H NMR (400 MHz, CD₃OD) δ 8.22 (s, 1 H), 5.89 (d, *J* = 6.0 Hz, 1 H), 4.66 (t, *J* = 5.2 Hz, 1 H), 4.53-4.49 (m, 1 H), 4.31 (t, *J* = 3.2 Hz, 1 H), 4.15-4.13 (m, 1 H), 3.88 (dd, *J* = 12.4 Hz, *J* = 2.4 Hz, 1 H), 4.74 (dd, *J* = 12.4 Hz, *J* = 4.0 Hz, 1 H), 2.11-2.05 (m, 2 H), 1.82-1.76 (m, 2 H), 1.73-1.58 (m, 4 H).

### Step 2) Preparation of ((3aR,4R,6R,6aR)-6-(2-chloro-6-(cyclopentylamino)-9H-purin-9-yl)-2,2-dimethyltetrahydrofuro[3,4-d][1,3]dioxol-4-yl)methanol (1-d)

**1-c** (4.0 g, 10.8 mmol), acetone (40 mL), 2,2-dimethoxypropane (5.637 g, 54.2 mmol), and PTSA (0.372 g, 2.16 mmol) were sequentially added to a 100 mL three-necked flask, and the system was purged with nitrogen gas three times and heated at reflux for 1 h. Analysis showed that the reaction was complete, and heating was stopped. The reaction mixture was cooled to room temperature (20 °C) and concentrated under reduced pressure to give an oil. Dichloromethane (150 mL) was added, and the mixture was extracted and washed with saturated sodium bicarbonate (60 mL × 2) and saturated brine (50 mL) in sequence, dried over anhydrous sodium sulfate (5 g), filtered, and concentrated to give 4.0 g of a yellow solid crude product. The crude product was directly used in the next step. Yield: 96.6%.

MS-ESI: 410.2[M+H]⁺.

¹H NMR (400 MHz, CD₃OD) δ 8.22 (s, 1 H), 6.09 (d, *J* = 3.6 Hz, 1 H), 5.23 (dd, *J* = 6.0 Hz, *J* = 3.2 Hz, 1 H), 5.01 (dd, *J* = 6.4 Hz, *J* = 2.4 Hz, 1 H), 4.53-4.49 (m, 1 H), 4.34 (dd, *J* = 6.0 Hz, *J* = 4.0 Hz, 1 H), 3.78 (dd, *J* = 12.0 Hz, *J* = 4.0 Hz, 1 H), 3.71 (dd, *J* = 12.0 Hz, *J* = 4.0 Hz, 1 H), 2.16-2.04 (m, 2 H), 1.83-1.76 (m, 2 H), 1.72-1.56 (m, 7 H), 1.37 (s, 3 H).

### Step 3) Preparation of ((3aR,4R,6R,6aR)-6-(2-chloro-6-(cyclopentylamino)-9H-purin-9-yl)-2,2-dimethyltetrahydrofuro[3,4-d][1,3]dioxol-4-yl)methyl pentadecanoate (1-e)

DCM (6 mL), **1-d** (600 mg, 1.46 mmol), DCC (453 mg, 2.2 mmol), and DMAP (18 mg, 0.146 mmol) were sequentially added to a 25 mL single-necked flask, and the system was purged with nitrogen gas three times and stirred at room temperature (19-26 °C) for 5 h. Analysis showed that the reaction was complete, and the reaction was stopped. The reaction mixture was filtered, and the filter cake was washed with dichloromethane twice, 20 mL each time. The filtrate was concentrated under reduced pressure to give an oil, and the oil was purified by column chromatography (EA/PE = 0-1/1) to give a white solid (896 mg, yield: 96%).

¹H NMR (400 MHz, CD₃OD) δ 8.13 (s, 1 H), 6.13 (d, *J* = 2.0 Hz, 1 H), 5.48-5.44 (m, 1 H), 5.43 (s, 1 H), 5.08-5.05 (m, 1 H), 4.53-4.48 (m, 1 H), 4.45-4.42 (m, 1 H), 4.27-4.25 (m, 2 H), 2.23-2.18 (m, 2 H), 2.11-2.04 (m, 2 H), 1.82-1.78 (m, 2 H), 1.72-1.58 (m, 6 H), 1.50-1.47 (m, 2 H), 1.39 (s, 3 H), 1.35-1.23 (m, 20 H), 0.89 (t, *J* = 6.8 Hz, 3 H).

### Step 4) Preparation of ((2R,35,4R,5R)-5-(2-chloro-6-(cyclopentylamino)-9H-purin-9-yl)-3,4-dihydroxytetrahydrofuran-2-yl)methyl pentadecanoate (compound 1)

Dichloromethane (6 mL) and **1-e** (600 mg, 0.946 mmol) were sequentially added to a 50 mL three-necked flask, and the system was purged with nitrogen gas three times and cooled in an ice-water bath until the internal temperature was 5 °C. TFA (6 mL) was added dropwise over about 5 min, with the internal temperature not higher than 5 °C. The ice-water bath was removed, and the mixture was stirred at room temperature for 3 h. Analysis showed that the reaction was complete, and the reaction was stopped. The reaction mixture was concentrated under reduced pressure to give an oil. Saturated brine (60 mL) was added, and extraction was performed with dichloromethane (60 mL × 3). The organic phases were combined, dried over anhydrous sodium sulfate (5 g), filtered, and concentrated to give an oil. The oil was purified by column chromatography (MeOH/DCM = 0% to 10%) to give a white solid (289 mg, yield: 51%).

MS-ESI: 594.3 [M+H]⁺.

¹H NMR (400 MHz, CDCI₃) δ 7.87 (s, 1 H), 6.01 (d, *J* = 3.2 Hz, 1 H), 5.90 (d, *J* = 5.2 Hz, 1 H), 5.43 (s, 1 H), 4.58-4.57 (m, 1 H), 4.54 (t, *J* = 5.2 Hz, 1 H), 4.52-4.47 (m, 1 H), 4.46-4.37 (m, 2 H), 4.28 (dd, *J* = 12.4 Hz, *J* = 4.0 Hz, 1 H), 3.26 (d, *J* = 1.6 Hz, 1 H), 2.28-2.20 (m, 2 H), 2.18-2.10 (m, 2 H), 1.57-1.43 (m, 2 H), 1.51-1.48 (m, 4 H), 1.31-1.22 (m, 22 H), 0.87 (t, *J* = 6.8 Hz, 3 H).

### Example 2: ((2R,3S,4R,5R)-5-(2-Chloro-6-(cyclopentylamino)-9H-purin-9-yl)-3,4-dihydroxytetrahydrofuran-2-yl)methyl benzoate (Compound 2)

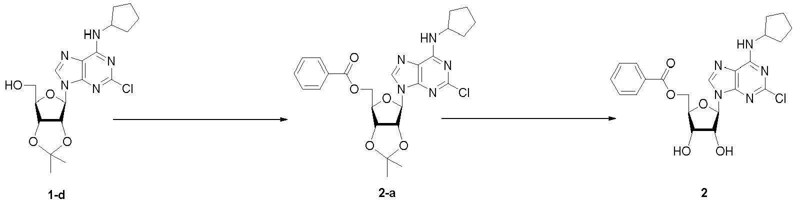

### Step 1) Preparation of ((3aR,4R,6R,6aR)-6-(2-chloro-6-(cyclopentylamino)-9H-purin-9-yl)-2,2-dimethyltetrahydrofuro[3,4-d][1,3]dioxol-4-yl)methyl benzoate (2-a)

In a nitrogen atmosphere, **1-d** (550 mg, 1.34 mmol, 1.0 eq) was added to DCM (25 mL) and stirred until the compound dissolved, and benzoic acid (196 mg, 1.74 mmol, 1.3 eq), DMAP (16.5 mg, 0.134 mmol, 0.1 eq), and DCC (418 mg, 2.01 mmol, 1.5 eq) were sequentially added. The mixture was left to react for 3-4 h, and in-process monitoring showed that the reaction was complete. The reaction mixture was directly concentrated under reduced pressure and then purified by reversed-phase column chromatography (ACN:H₂O (1% TFA) = 0-95%) to give **2-a** (610 mg, yield: 88%).

MS-ESI: 514.2 [M+H]⁺.

### Step 2) Preparation of ((2R,3S,4R,5R)-5-(2-chloro-6-(cyclopentylamino)-9H-purin-9-yl)-3,4-dihydroxytetrahydrofuran-2-yl)methyl benzoate (compound 2)

**2-a** (610 mg, 1.1 mmol, 1.0 eq) was dissolved in DCM (3 mL), and TFA (3 mL) was then added. The mixture was stirred in a nitrogen atmosphere for 2-3 h, and in-process monitoring showed that the reaction was complete. The reaction mixture was concentrated under reduced pressure to remove the solvent, and the residue was dissolved in DCM (30 mL). Then the solution was washed with saturated sodium bicarbonate (40 mL) and concentrated under reduced pressure, and the residue was purified by column chromatography (EA:PE = 0-75%) to give **compound 2** (130 mg, yield: 23.11%).

MS-ESI: 474.1 [M+H]⁺.

¹H NMR (400 M, CDCl₃): δ 7.86-7.84 (m, 3H), 7.52 (t, *J* = 7.6 Hz, 1H), 7.38-7.26 (m, 2H), 6.10-6.08 (bs, 1H), 5.91 (d, *J* = 7.6 Hz, 1H), 5.48 (s, 1H), 4.72-4.47 (m, 6H), 3.26-3.23 (m, 1H), 2.22-2.17 (m,2H), 1.77-1.72 (m, 2H), 1.55-1.53 (m,2H), 1.33-1.31 (m, 1H).

### Example 3: ((2R,3S,4R,5R)-5-(2-Chloro-6-(cyclopentylamino)-9H-purin-9-yl)-3,4-dihydroxytetrahydrofuran-2-yl)methyl isobutyrate (Compound 3)

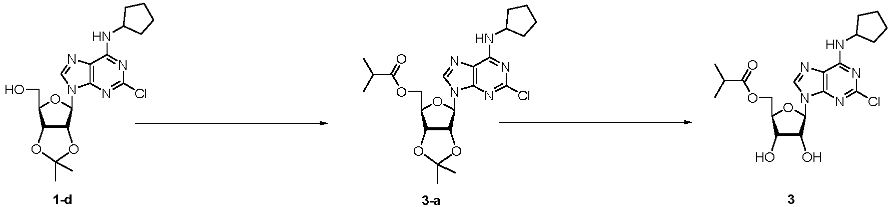

### Step 1) Preparation of ((3aR,4R,6R,6aR)-6-(2-chloro-6-(cyclopentylamino)-9H-purin-9-yl)-2,2-dimethyltetrahydrofuro[3,4-d][1,3]dioxol-4-yl)methyl isobutyrate (3-a)

In a nitrogen atmosphere, **1-d** (550 mg, 1.34 mmol, 1.0 eq) was added to DCM (25 mL) and stirred, and then isobutyric acid (154 mg, 1.74 mmol, 1.3 eq), DMAP (16.5 mg, 0.134 mmol, 0.1 eq), and DCC (418 mg, 2.01 mmol, 1.5 eq) were sequentially added. The mixture was left to react for 3-4 h, and in-process monitoring showed that the reaction was complete. The reaction mixture was directly concentrated under reduced pressure and then purified by reversed-phase column chromatography (ACN:H₂O (1% TFA) = 0-95%) to give **3-a** (613 mg, yield: 95%).

MS-ESI: 480.2 [M+H]⁺.

### Step 2) Preparation of ((2R,3S,4R,5R)-5-(2-chloro-6-(cyclopentylamino)-9H-purin-9-yl)-3,4-dihydroxytetrahydrofuran-2-yl)methyl isobutyrate (compound 3)

In a nitrogen atmosphere, **3-a** (610 mg, 1.1 mmol, 1.0 eq) was dissolved in DCM (3 mL), and TFA (3 mL) was then added. The mixture was stirred for 2-3 h, and in-process monitoring showed that the reaction was complete. The reaction mixture was concentrated under reduced pressure to remove the solvent, and the residue was dissolved in DCM (30 mL). Then the solution was washed with saturated sodium bicarbonate (40 mL) and concentrated under reduced pressure, and the residue was purified by column chromatography (EA:PE = 0-75%) to give **compound 3** (291 mg, yield: 23.11%).

MS-ESI: 440.2 [M+H]⁺.

¹H NMR (400 MHz, CDCl₃) δ 7.89 (s, 1H), 6.10 (bs, 1H), 5.92 (d, *J* = 4.8Hz, 1H), 5.54 (bs, 1H), 4.58-4.108 (m, 6H), 3.38 (bs, 1H), 2.55-2.48 (m, 1H), 2.17-2.10 (m, 2H), 1.80-1.66 (m, 7H), 1.57-1.53 (m, 2H), 1.13-0.96 (m, 3H).

### Example 4: ((2R,3S,4R,5R)-5-(2-Chloro-6-(cyclopentylamino)-9H-purin-9-yl)-3,4-dihydroxytetrahydrofuran-2-yl)methyl L-valinate (Compound 4)

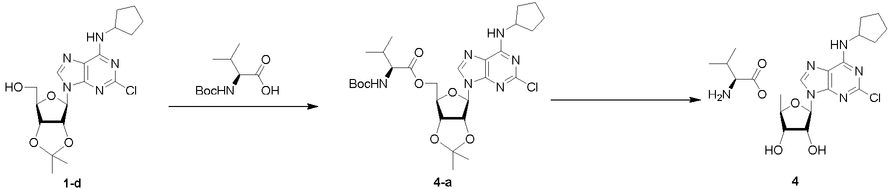

### Step 1) Preparation of ((3aR,4R,6R,6aR)-6-(2-chloro-6-(cyclopentylamino)-9H-purin-9-yl)-2,2-dimethyltetrahydrofuro[3,4-d][1,3]dioxol-4-yl) L-valine (4-a)

DCM (10 mL), **1-d** (500 mg, 1.22 mmol), Boc-D-valine (345 mg, 1.59 mmol), DCC (377 mg, 1.83 mmol), and DMAP (15 mg, 0.122 mmol) were sequentially added to a 25 mL single-necked flask, and the system was purged with nitrogen gas three times and stirred at room temperature (19-26 °C) for 5 h. Analysis showed that the reaction was complete, and the reaction was stopped. The reaction mixture was filtered, and the filter cake was washed with dichloromethane twice, 20 mL each time. The filtrate was concentrated under reduced pressure to give an oil, and the oil was purified by column chromatography (EA/PE = 0:1-1:1) to give a white solid (718 mg, yield: 96%).

MS-ESI: 609.3 [M+1]⁺.

### Step 2) Preparation of ((2R,3S,4R,5R)-5-(2-chloro-6-(cyclopentylamino)-9H-purin-9-yl)-3,4-dihydroxytetrahydrofuran-2-yl)methyl L-valinate (compound 4)

TFA (6 mL) and **1-d** (600 mg, 0.946 mmol) were sequentially added to a 50 mL three-necked flask, and the system was purged with nitrogen gas three times and stirred at room temperature (19-26 °C) for 2 h. Analysis showed that the reaction was complete. The reaction mixture was concentrated under reduced pressure to give an oil. Saturated sodium bicarbonate (60 mL) was added, and extraction was performed with dichloromethane (60 mL × 3). The organic phases were combined, dried over anhydrous sodium sulfate (5 g), filtered, and concentrated to give an oil. The oil was purified by column chromatography (MeOH/DCM = 0%-10%) to give a white solid (206 mg, yield: 44.6%).

MS-ESI: 469.2 [M+1]⁺.

¹H NMR (400 MHz, CD₃OD) δ 8.16 (s, 1 H), 5.92 (d, *J* = 4.4 Hz, 1 H), 4.78 (t, *J* = 4.8 Hz, 1 H), 4.51-4.46 (m, 1 H), 4.44-4.38 (m, 3 H), 4.27-4.23 (m, 1 H), 2.10-2.05 (m, 2 H), 1.97-1.92 (m, 1 H), 1.82-1.76 (m, 2 H), 1.73-1.35 (m, 4 H), 0.91 (d, *J* = 7.2 Hz, 3 H), 0.87 (d, *J=* 6.8 Hz, 3 H).

### Biological Evaluations

### Test Example 1

### 1. Exprimental materials

| **Reagent/instrument** | **Manufacturer** | **Cat. No./Specification** |
|---|---|---|
| Complete Freund's adjuvant (CFA) | Sigma-Aldrich, St. Louis, MO | F5881 |
| 0.9% sodium chloride injection | Anhui Double-Crane Pharmaceutical Co., Ltd. | 500 mL |
| 2-Chloro-N6-cyclopentyladenosine (CCPA) | Sigma-Aldrich, St. Louis, MO | 37739-05-2 |
| VonFrey electronic aesthesiometer | Bioseb, Vitrolles, France | BIO-EVF4 |

**Experimental animals:** SPF male Sprague-Dawley (SD) rats, weighing 180±30 g, obtained from Shanghai Sippe-Bk Lab Animal Co., Ltd.

**Experimental samples:** Compounds 1, 2, and 3 and CCPA were used to prepare the corresponding pharmaceutical compositions according to the following formulation amounts and method.

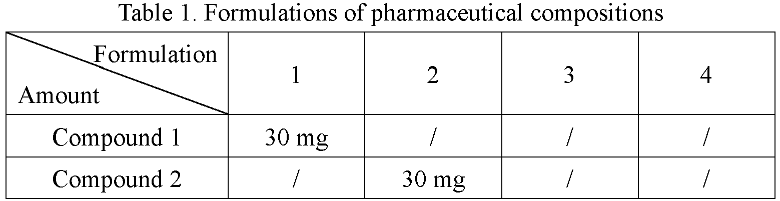

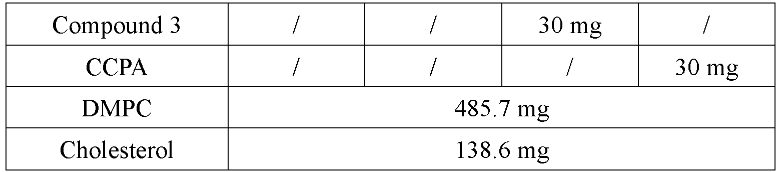

Preparation method: Formulation amounts of compounds 1, 2, and 3 and CCPA were separately weighed out, added to a proper amount of tert-butanol/water mixed solution, and dissolved by stirring. Then formulation amounts of dimyristoylphosphatidylcholine (DMPC) and cholesterol were added and dissolved by stirring.

The above solutions were made up to volume (25-40 mL), added to vials, and lyophilized in a lyophilizer for later use. When used, the lyophilizates were dissolved in normal saline to 1 mg/mL, 2 mg/mL, 4 mg/mL, and 8 mg/mL.

### 2. Experimental method

### 2.1. Animals and administration

SD rats, 6 to 8-week-old and weighing 150 g-200 g, were divided into a control group, a model control group, and an administration group. A CFA inflammatory pain model was obtained 24 h after 100 µL of complete Freund's adjuvant was injected into the soles of the left feet of the rats in the model control group and the administration group.

The injection was performed at left Zusanli, and the injection volume was 100 µL.

Specific administration information is shown in Table 2.

**Table 2. Administration information**

| Group | Route of administration | Concentration (mg/mL) | Volume (100 µL) | Number of animals (rats) |
|---|---|---|---|---|
| Control group | Injection | - | - | 6 |
| Model control group | | - | 100 | 6 |
| Administration group | | 1 mg/mL | 100 | 6 |
| | | 2 mg/mL | 100 | 6 |
| | | 4 mg/mL | 100 | 6 |
| | | 8 mg/mL | 100 | 6 |

| | | | | |
|---|---|---|---|---|
| Note: For the control group, 100 µL of normal saline was injected into the soles of the feet. For the model control group, 100 µL of normal saline was injected into Zusanli after the model was successfully established. | | | | |

### 2.2. Evaluation indices and data

A VonFrey electronic aesthesiometer was used to measure the pain threshold, and mechanical pain was used as an evaluation index. Observations of the basal pain threshold were started 1 day before the model was established and ended 14 days after the model was established.

Pain thresholds before and after administration for different groups of animals were plotted using Graph Pad Prism 8.3.0 software. Based on the dose-response curve 48 h after administration, the EC₅₀ and EC₉₀ were obtained through fitting.

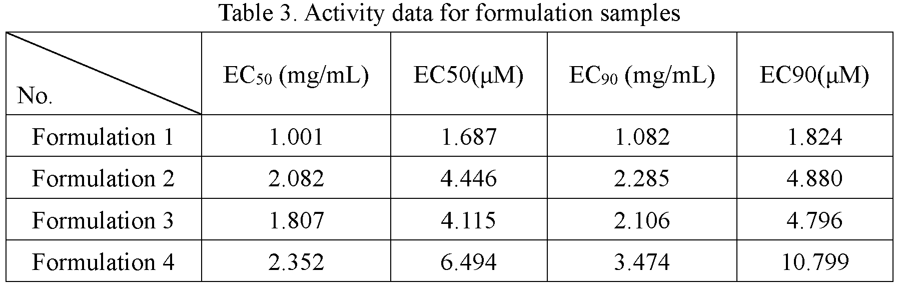

Conclusion: Biological activity data show that after "fatty alkanoyl" modification at the 5' site, the EC₅₀ and EC₉₀ values of compound 1 significantly decreased, and increased 4-fold and 6-fold, respectively, compared to CCPA. In addition, compared to compound 2, the EC₅₀ and EC₉₀ also increased nearly 3-fold. Unlike the prior art (or common sense), the activity of the fatty compound is comparable to or worse than that of the compounds.

## Claims

1. A compound represented by formula (I) or a pharmaceutically acceptable salt thereof, 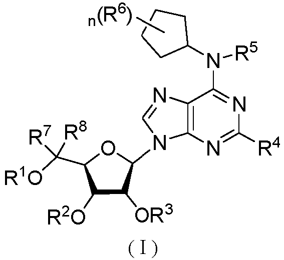 wherein:
R¹, R², and R³ are identical or different and are each independently selected from the group consisting of hydrogen, 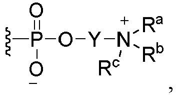 -(CO)-C₁₋₂₀ alkyl, -(CO)-C₁₋₂₀ alkoxy, - (CO)-5-6 membered cycloalkyl, -(CO)-5-6 membered heterocyclyl, -(CO)-6-membered aryl, and -(CO)-5-6 membered heteroaryl, wherein the alkyl, alkoxy, cycloalkyl, heterocyclyl, aryl, or heteroaryl is optionally substituted with one or more substituents selected from the group consisting of halogen, hydroxy, oxo, amino, cyano, C₁₋₆ alkyl, and C₁₋₆ alkoxy;
Y is selected from the group consisting of alkylene and heteroalkylene, wherein the alkylene or heteroalkylene is optionally substituted with one or more substituents selected from the group consisting of halogen, hydroxy, oxo, amino, cyano, C₁₋₆ alkyl, and C₁₋₆ alkoxy;
R^{a}, R^{b}, and R^{c} are identical or different and are each independently C₁₋₆ alkyl,
and R¹, R², and R³ cannot be hydrogen at the same time;
R⁴ is selected from the group consisting of halogen, hydroxy, amino, cyano, C₁₋₆ alkyl,
and C₁₋₆ alkoxy, wherein the alkyl or alkoxy is optionally substituted with one or more substituents selected from the group consisting of halogen, hydroxy, amino, cyano, C₁₋₆ alkyl, and C₁₋₆ alkoxy;
R⁵, R⁶, R⁷, and R⁸ are identical or different and are each independently selected from the group consisting of hydrogen, halogen, hydroxy, amino, cyano, C₁₋₆ alkyl, and C₁₋₆ alkoxy, wherein the alkyl or alkoxy is optionally substituted with one or more substituents selected from the group consisting of halogen, hydroxy, amino, cyano, C₁₋₆ alkyl, and C₁₋₆ alkoxy;
n is selected from the group consisting of 0, 1, 2, 3, 4, 5, 6, 7, and 8.

2. The compound or the pharmaceutically acceptable salt thereof according to claim 1, wherein both R² and R³ are hydrogens.

3. The compound or the pharmaceutically acceptable salt thereof according to claim 1 or 2, wherein the compound represented by formula (I) or the pharmaceutically acceptable salt thereof is a compound represented by formula (I-1) or a pharmaceutically acceptable salt thereof, 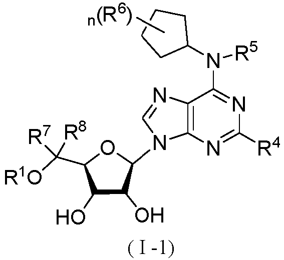 wherein R¹, R⁴, R⁵, R⁶, R⁷, R⁸, and n are as defined in claim 1.

4. The compound or the pharmaceutically acceptable salt thereof according to any one of claims 1-3, wherein R⁴ is halogen, preferably fluorine, chlorine, or bromine, and most preferably chlorine.

5. The compound or the pharmaceutically acceptable salt thereof according to any one of claims 1-4, wherein the compound represented by formula (I) or the pharmaceutically acceptable salt thereof is a compound represented by formula (I-2) or a pharmaceutically acceptable salt thereof, 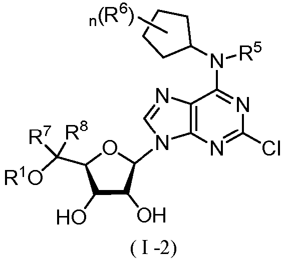 wherein R¹, R⁵, R⁶, R⁷, R⁸, and n are as defined in claim 1.

6. The compound or the pharmaceutically acceptable salt thereof according to any one of claims 1-5, wherein R⁵ is hydrogen.

7. The compound or the pharmaceutically acceptable salt thereof according to any one of claims 1-6, wherein R⁶ is hydrogen.

8. The compound or the pharmaceutically acceptable salt thereof according to any one of claims 1-7, wherein the compound represented by formula (I) or the pharmaceutically acceptable salt thereof is a compound represented by formula (I-3) or a pharmaceutically acceptable salt thereof, 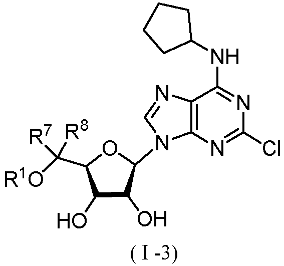 wherein R¹, R⁷, and R⁸ are as defined in claim 1.

9. The compound or the pharmaceutically acceptable salt thereof according to any one of claims 1-8, wherein both R⁷ and R⁸ are hydrogens.

10. The compound or the pharmaceutically acceptable salt thereof according to any one of claims 1-9, wherein the compound represented by formula (I) or the pharmaceutically acceptable salt thereof is a compound represented by formula (I-4) or a pharmaceutically acceptable salt thereof, 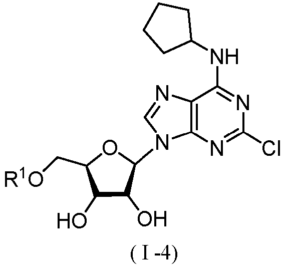 wherein R¹ is as defined in claim 1.

11. The compound or the pharmaceutically acceptable salt thereof according to any one of claims 1-10, wherein R¹ is selected from the group consisting of -(CO)-C₁₋₅ alkyl, - (CO)-C₁₀₋₂₀ alkyl, -(CO)-5-6 membered cycloalkyl, -(CO)-5-6 membered heterocyclyl, - (CO)-phenyl, -(CO)-5-6 membered heteroaryl, and 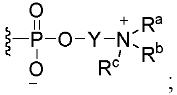
Y is C₁₋₆ alkylene;
R^{a}, R^{b}, and R^{c} are identical or different and are each independently C₁₋₆ alkyl;
the alkylene, alkyl, cycloalkyl, heterocyclyl, phenyl, or heteroaryl is optionally substituted with one or more substituents selected from the group consisting of halogen, hydroxy, amino, cyano, oxo, C₁₋₆ alkyl, and C₁₋₆ alkoxy, and the substituents are preferably fluorine, chlorine, bromine, hydroxy, amino, oxo, and methyl.

12. The compound or the pharmaceutically acceptable salt thereof according to claim 11, wherein R¹ is selected from the group consisting of -(CO)-methyl, -(CO)-ethyl, -(CO)-propyl, -(CO)-butyl, -(CO)-pentyl, -(CO)-C₁₄ alkyl, -(CO)-phenyl, -(CO)-cyclopropane, -(CO)-cyclobutane, -(CO)-cyclopentane, -(CO)-cyclohexane, -(CO)-pyrrolidine, -(CO)-piperidine, -(CO)-piperazine, -(CO)-morpholine, -(CO)-thiomorpholine, -(CO)-pyrrole, - (CO)-furan, -(CO)-thiophene, -(CO)-pyridine, -(CO)-thiazole, -(CO)-oxazole, -(CO)-imidazole, -(CO)-triazole, -(CO)-tetrazole, and 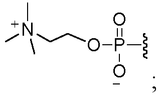 the R¹ is optionally substituted with one or more substituents selected from the group consisting of halogen, hydroxy, amino, cyano, oxo, C₁₋₆ alkyl, and C₁₋₆ alkoxy, and the substituents are preferably fluorine, chlorine, bromine, hydroxy, amino, oxo, and methyl.

13. The compound or the pharmaceutically acceptable salt thereof according to any one of claims 1-12, wherein the compound or the pharmaceutically acceptable salt thereof is selected from the group consisting of any one of the compounds in the table below,

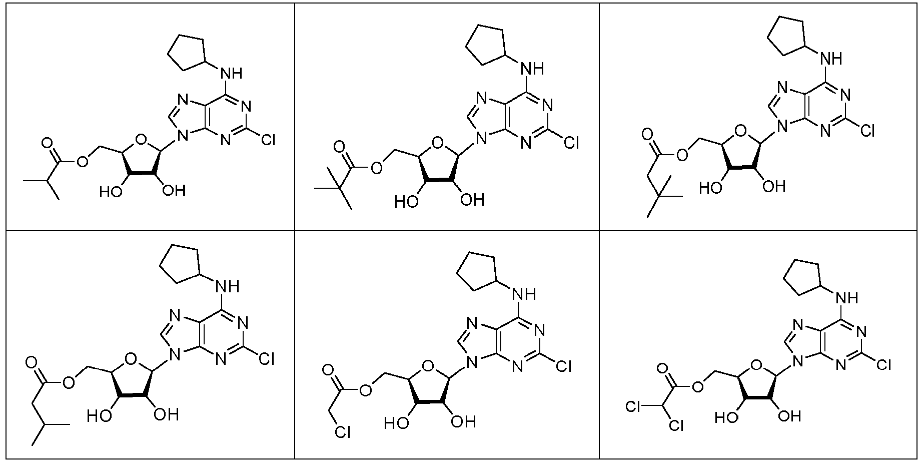

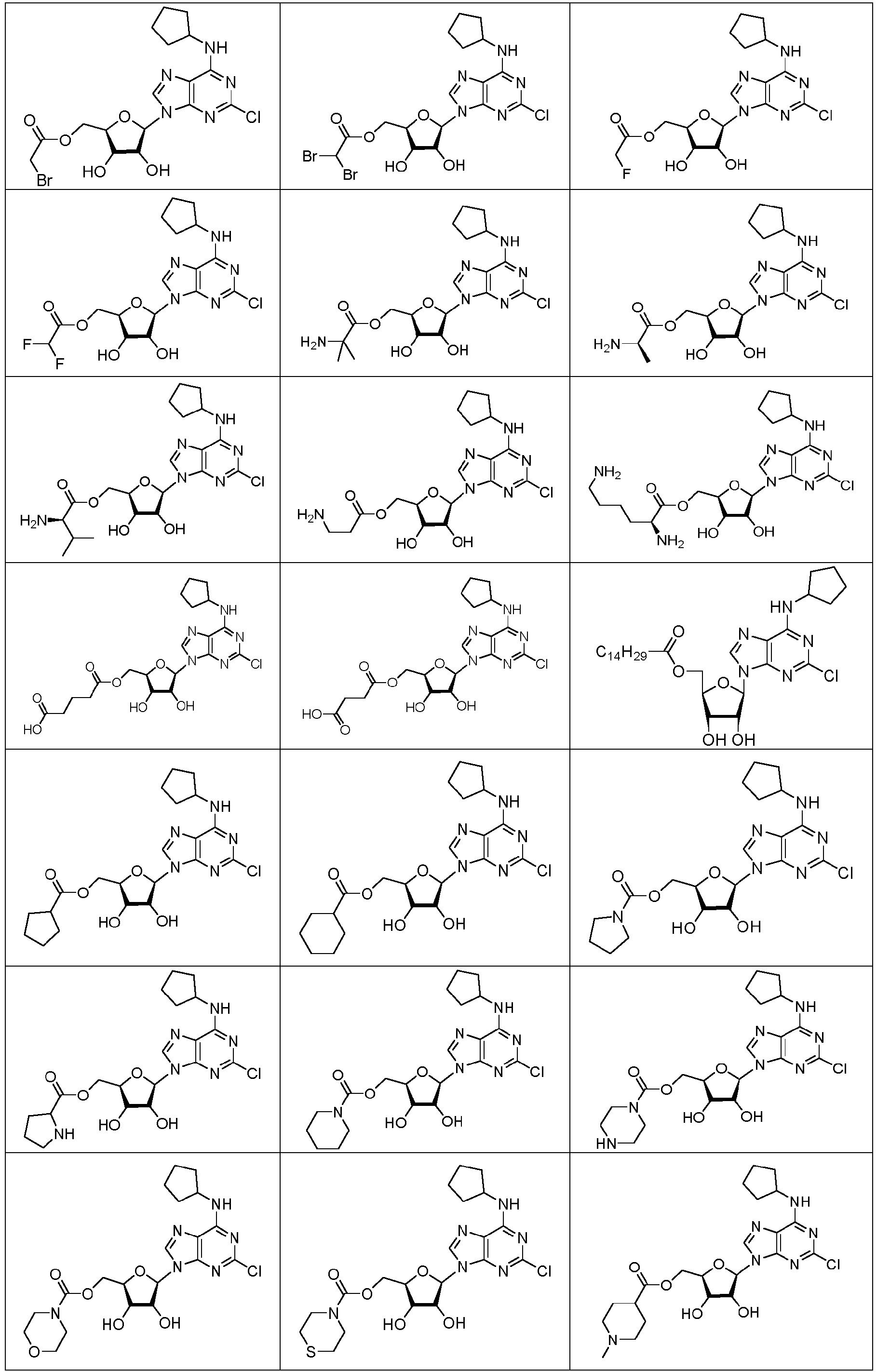

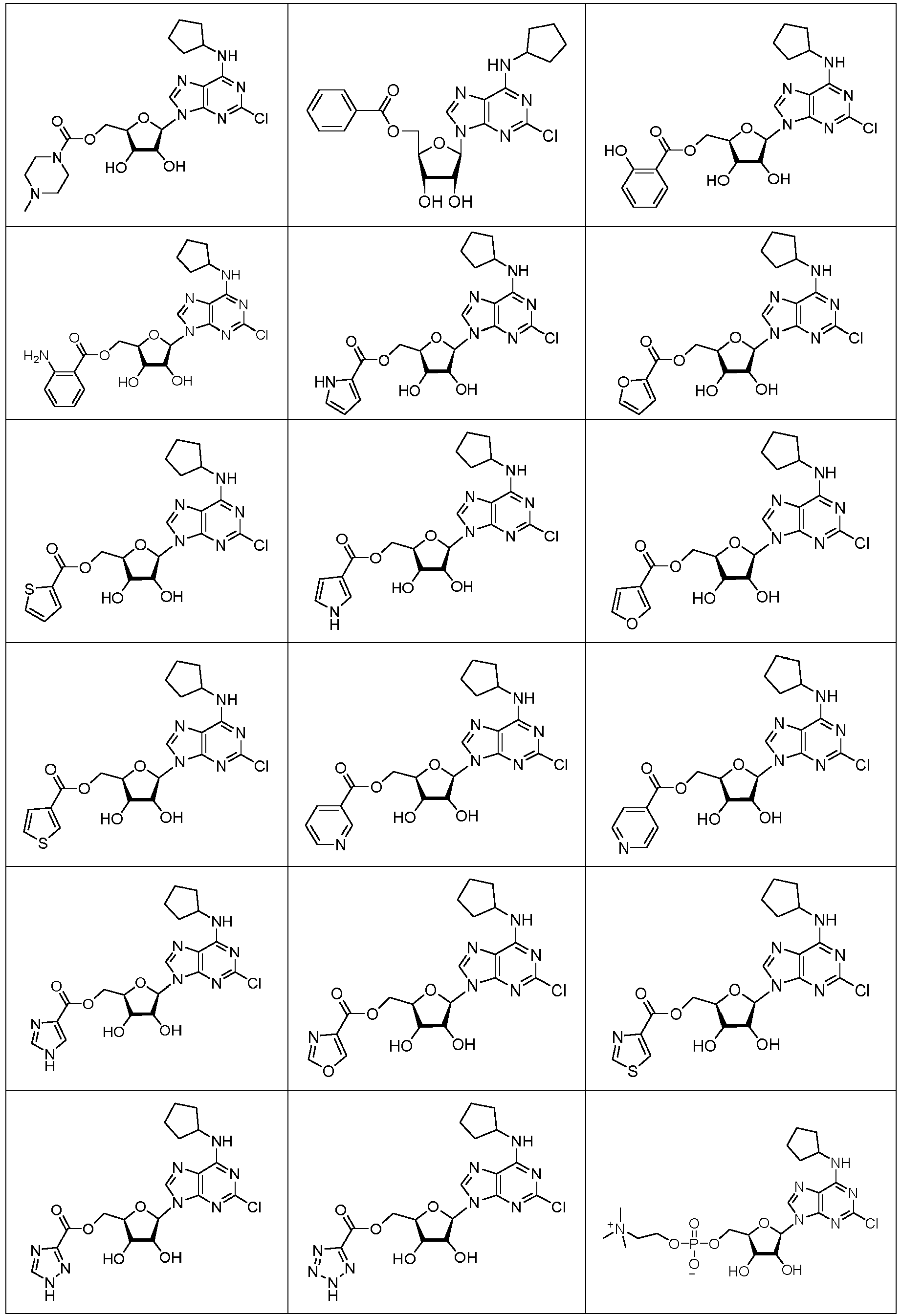

14. An isotopically substituted form of the compound or the pharmaceutically acceptable salt thereof according to claim 13, wherein preferably, the isotopically substituted form is a deuterated form.

15. A pharmaceutical composition, comprising a therapeutically effective amount of at least one of the compound or the pharmaceutically acceptable salt thereof according to any one of claims 1-13 or the isotopically substituted form according to claim 14 and a pharmaceutically acceptable excipient.

16. A method for treating or preventing chronic pain, comprising administering to a patient a therapeutically effective amount of the compound according to any one of claims 1-13, or the isotopically substituted form according to claim 14, or the pharmaceutical composition according to claim 15.

17. Use of the compound according to any one of claims 1-13, or the isotopically substituted form according to claim 14, or the pharmaceutical composition according to claim 15 in the preparation of a medicament for treating or preventing chronic pain.
